# EUROPEAN PATENT APPLICATION

(11) **EP 4 617 008 A1**
(43) Date of publication of application: **17.09.2025**
(21) Application number: 24188380.0
(22) Date of filing: 12.07.2024
(51) Int. Cl.: B25J 9/16, G06F 30/20, G06N 3/126, G05B 19/418, G06F 111/06, G06F 113/18, G06F 115/12, G06F 119/18, H01L 21/67

(54) **OPTIMIZATION SYSTEM FOR PROCESS OF COMPONENT RETRIEVAL AND PLACEMENT AND METHOD THEREFOR**

(30) Priority: 12.03.2024 CN 202410278536
(71) Applicant: Delta Electronics, Inc., Taoyuan County 33341 (TW)
(72) Inventor: Yeh, Jou Chun, 33341 Taoyuan City (TW); Yeh, Yu Wei, 33341 Taoyuan City (TW); Lu, Shao Huang, 33341 Taoyuan City (TW); Kung, Ju Hsin, 33341 Taoyuan City (TW)
(74) Representative: Petraz, Gilberto Luigi

(57) **Abstract**

An optimization system for component retrieval and placement and a method therefor are provided. The optimization system processes multiple parameters inputted by a user through an optimization calculation module (11), and processes the calculated solutions through the optimization calculation module (11) to output multiple final parameters. The optimization calculation module (11) utilizes a genetic algorithm and integrates a precision-designed penalty mechanism, allowing the user to only provide specific constraints and retrieval components that need to be optimized, to ensure that the optimal machine layout as well as the retrieval and placement sequence are found under the given constraints.

## Description

### FIELD OF INVENTION

The present disclosure relates to the technical field of object retrieval and placement, and in particular, relates to an optimization system for process of component retrieval and placement.

### BACKGROUND OF INVENTION

There are many automation application fields involving object retrieval and placement, such as package retrieval and placement in the warehousing and logistics industry, component retrieval and placement in the electronic assembly industry, and medicine retrieval, placement and distribution in the medical industry. In other fields such as manufacturing, food processing and packaging, there are application situations for object retrieval and placement.

In the field of electronic assembly, the manufacturing of printed circuit boards (PCB) requires the use of automatic insertion machines to automatically install electronic components on printed circuit boards according to designed procedures. However, normal insertion machines primarily rely on experts to manually plan the program for the feeding strategy and retrieval and placement sequence of these electronic components. In terms of feeding strategy, experts must consider the placement configuration of components in feeding mechanisms and appropriately select matching pick-up mechanisms. This selection involves multiple mechanical constraints. For example, some pick-up mechanisms can only be used with specific feeding mechanisms, or certain points for inserting components can only be reached by specific pick-up mechanisms. In addition, since different types of components may vary in size, a single pick-up mechanism can only handle the same type of components in each complete insertion process. Therefore, experts must consider these limitations during the planning process.

In terms of planning the component retrieval and placement sequence, experts need to pay special attention to the component retrieval sequence and the insertion point sequence, since these factors will directly affect the insertion cycle time and overall operation smoothness. Once the planning is finished, experts must input the information into a simulation environment to calculate insertion times and select a configuration combination with the best performance based on the results of multiple rounds of simulations. It is noted that these planning results only apply to specific types of printed circuit boards. Once a different type of printed circuit boards is designed, since the points for arranging components may vary, experts would need to re-plan for new cases. This process is not only time-consuming, but also requires considerable efforts.

Therefore, determining the optimal machine layout as well as the retrieval and placement sequence based on the machines and the environment, so as to achieve a more efficient and economical production process, has become one of the problems that need to be solved in this field.

### SUMMARY OF INVENTION

In order to achieve a more efficient and economical production process, the present disclosure provides a method and a system that can optimize the component retrieval and placement sequence and configuration based on user-defined constraints. The system comprises an optimization calculation module which focuses on determining the component retrieval and placement sequence and configuration based on user-defined constraints, and integrates a penalty mechanism. Users merely need to provide specific constraints and data of retrieval components that need to be optimized, to ensure that the optimal machine layout as well as the retrieval and placement sequence are found under the given constraints.

One object of the present disclosure is to provide a method for optimizing the process of component retrieval and placement. The method is executed by an optimization calculation module and comprises:
(a) receiving multiple parameters and at least one constraint, wherein the multiple parameters and the at least one constraint are associated with at least one of feeding mechanisms, pick-up mechanisms, types of components and component retrieval and placement sequences;
(b) executing a genetic algorithm based on the multiple parameters and the at least one constraint; and
(c) outputting multiple final parameters representing optimized component retrieval and placement sequences and configurations based on results obtained via the execution of the genetic algorithm;
wherein the genetic algorithm comprises (i) encoding to randomly generate multiple chromosomes according to the multiple parameters, and the multiple chromosomes forming an initial chromosome pool; (ii) decoding the multiple chromosomes in the initial chromosome pool based on the at least one constraint; (iii) performing an evaluation on the decoded chromosomes to obtain fitness scores, and the evaluation includes a feedback of a penalty value when the decoded chromosomes violate the at least one constraint; and (iv) selecting a chromosome with the smallest fitness score, and generating a chromosome that represents an optimized component retrieval and placement sequence and configuration through multiple iterations and selections.

In the embodiments of the present disclosure, the method further comprises (b1): simulating an actual physical movement of a machine based on the decoded chromosomes to estimate a production cycle time for the decoded chromosomes after step (b) and prior to step (c).

In the embodiments of the present disclosure, the method further comprises (c1): presenting the multiple final parameters in a diagram after step (c).

In the embodiments of the present disclosure, the at least one constraint comprises a configuration sequence of the feeding mechanisms, correspondences between the pick-up mechanisms and the components to be picked, and a quantity limit of the components on the feeding mechanisms.

In the embodiments of the present disclosure, the evaluation is performed based on values of M, F, D, weight 1, and weight 2 to obtain the fitness scores, wherein M represents a constant value for the evaluation, weight 1 represents the penalty value, F represents the quantity that exceeds the at least one constraint, weight 2 represents a distance weight of the decoded chromosome, and D represents a distance.

In the embodiments of the present disclosure, the multiple iterations and selections comprises selecting at least two chromosomes with the smallest value based on the multiple parameters for mutual mating and mutation to generate new chromosome filial generations; performing the evaluation on the new chromosome filial generations, selecting at least two chromosome filial generations with the smallest value from the new chromosome filial generations for another mutual mating and mutation to generate the next new filial generations of the chromosome filial generations; and taking a chromosome with the smallest value as the chromosome representing the optimized component retrieval and placement sequence and configuration when the number of iterations exceeds a default value.

In the embodiments of the present disclosure, the default value can be, for example, between 10 and 100. Preferably, the default value is 20 to 50, and more preferably is 30, which represents that the process of mating, mutation and producing filial generations can be repeated 30 times.

In the embodiments of the present disclosure, the multiple parameters comprise candidate positions of the feeding mechanisms, end point information of the pick-up mechanisms, positions of the placing points, and physical limitations of actuators.

In the embodiments of the present disclosure, the multiple parameters further comprise correspondences between the pick-up mechanisms and the components, a configuration sequence of specific feeding mechanisms, and a quantity range of the specific components on the feeding mechanisms.

In the embodiments of the present disclosure, the multiple final parameters comprise a total quantity of the feeding mechanisms, types of the components and configuration of the feeding mechanisms, configuration of the pick-up mechanisms and the feeding mechanisms, configuration of the pick-up mechanisms and the components, component retrieval and placement sequence, a predicted cycle time, a relation diagram of initial components and mechanisms, placing configuration and usage frequency distribution diagram of the feeding position, a path diagram of the component retrieval and placement, and a distribution diagram of a path length.

Another object of the present disclosure is to provide a system for optimizing the process of the component retrieval and placement. The system comprises an optimization calculation module configured to process multiple parameters and at least one constraint inputted by a user and output multiple final parameters representing optimized component retrieval and placement sequences and configurations based on results obtained via the processing, wherein the multiple parameters and the at least one constraint are associated with at least one of feeding mechanisms, pick-up mechanisms, types of components and component retrieval and placement sequences, and the optimization calculation module comprises:
an optimization core module, configured to execute a genetic algorithm;
wherein the genetic algorithm comprises (i) encoding to randomly generate multiple chromosomes according to the multiple parameters, and the multiple chromosomes forming an initial chromosome pool; (ii) decoding the multiple chromosomes in the initial chromosome pool based on the at least one constraint; (iii) performing an evaluation on the decoded chromosomes to obtain fitness scores, and the evaluation includes a feedback of a penalty value when the decoded chromosomes violate the at least one constraint; and (iv) selecting a chromosome with the smallest fitness score, and generating a chromosome that represents an optimized component retrieval and placement sequence and configuration through multiple iterations and the selections.

In the embodiments of the present disclosure, the optimization calculation module further comprises a data verification module connected to the optimization core module and configured to perform further evaluation on the chromosomes decoded by the optimization core module to accelerate the convergence of iterations.

In the embodiments of the present disclosure, the optimization calculation module further comprises a physics module connected to the optimization core module and configured to simulate an actual physical movement of a machine based on the decoded chromosomes to estimate a production cycle time for the decoded chromosomes.

In the embodiments of the present disclosure, the optimization calculation module further comprises a visualization module connected to the optimization core module and configured to present the multiple final parameters in a diagram.

In the embodiments of the present disclosure, the at least one constraint comprises a configuration sequence of the feeding mechanisms, correspondences between the pick-up mechanisms and the components to be picked, and a quantity limit of the components on the feeding mechanisms.

In the embodiments of the present disclosure, the evaluation is performed based on values of M, F, D, weight 1, and weight 2 to obtain the fitness scores, wherein M represents a constant value for the evaluation, weight 1 represents the penalty value, F represents the quantity that exceeds the at least one constraint, weight 2 represents a distance weight of the decoded chromosome, and D represents a distance.

In the embodiments of the present disclosure, the multiple iterations and selections comprises selecting at least two chromosomes with the smallest value based on the multiple parameters for mutual mating and mutation to generate new chromosome filial generations; performing the evaluation on the new chromosome filial generations, selecting at least two chromosome filial generations with the smallest value from the new chromosome filial generations for another mutual mating and mutation to generate the next new filial generations of the chromosome filial generations; and taking a chromosome with the smallest value as the chromosome representing the optimized component retrieval and placement sequence and configuration when the number of iterations exceeds a default value.

In the embodiments of the present disclosure, the default value can be, for example, between 10 and 100. Preferably, the default value is 20 to 50, and more preferably is 30, which represents that the process of mating, mutation and producing filial generations can be repeated 30 times.

In the embodiments of the present disclosure, the multiple parameters comprise candidate positions of the feeding mechanisms, end point information of the pick-up mechanisms, positions of the placing points, and physical limitations of actuators.

In the embodiments of the present disclosure, the multiple parameters further comprise correspondences between the pick-up mechanisms and the components, a configuration sequence of specific feeding mechanisms, and a quantity range of the specific components on the feeding mechanisms.

In the embodiments of the present disclosure, the multiple final parameters comprise a total quantity of the feeding mechanisms, types of the components and configuration of the feeding mechanisms, configuration of the pick-up mechanisms and the feeding mechanisms, configuration of the pick-up mechanisms and the components, component retrieval and placement sequence, a predicted cycle time, a relation diagram of initial components and mechanisms, placing configuration and usage frequency distribution diagram of the feeding position, a path diagram of the component retrieval and placement, and a distribution diagram of a path length.

Compared with related technologies, the advantages of the present disclosure are that:
(1) it can provide highly intuitive diagrams to allow users to quickly understand the optimal machine layout as well as the retrieval and placement sequence;
(2) the system can accurately predict the cycle time only based on evaluations, avoiding tedious and time-consuming actual simulations through the establishment of an actuator model, thereby significantly reducing the computational burden while maintaining prediction accuracy; and
(3) the system allows users to customize various constraints, which not only increases the flexibility of the optimization process, but also ensures that the final results comply with actual needs and rules, thereby significantly saving the time cost for experts to perform debugging and error testing.

The above advantages enable the system of the present disclosure to significantly save the time required for expert planning and personnel to adjust machine configurations, thereby improving overall production efficiency and economic benefits. The technology of the present disclosure can be applied in various fields. For example, in the field of surface mount technology (SMT), it can be used to optimize the component retrieval and placement and improve production speed and accuracy; in robotics, it can be used to guide robotic arms or other automatic equipment to perform more efficient and precise activities of retrieval and placement. Different industries and business situations can customize and apply the technology of the present disclosure according to their specific needs.

### DESCRIPTION OF DRAWINGS

FIGS. 1A and 1B are a flowchart of the system for optimizing the process of the component retrieval and placement of the present disclosure and a composition diagram of the optimization calculation module 11 in one embodiment of the system of the present disclosure, respectively.
FIG. 2 is an iterative flowchart of the genetic algorithm of the present disclosure.
FIGS. 3A and 3B are a cross-sectional view and a perspective view of the insertion processing equipment, respectively.
FIG. 4 illustrates a PCB with multiple types of insertion components and multiple insertion positions; and the goal is to insert the multiple types of components into the multiple insertion positions to minimize the total cycle time for completing the PCB insertion work.
FIG. 5 is a chart of the feeder configuration planned by the system for optimizing the process of the component retrieval and placement of the present disclosure in Situation I.
FIG. 6 is a chart of the feeder configuration planned by experts in Situation I.
FIG. 7 is a schematic diagram of the component placement sequences planned by the system for optimizing the process of the component retrieval and placement of the present disclosure in Situation I.
FIG. 8 is a schematic diagram of the component placement sequences planned by experts in Situation I.
FIG. 9 is a chart of the feeder configuration planned by the system for optimizing the process of the component retrieval and placement of the present disclosure in Situation II.
FIG. 10 is a chart of the feeder configuration planned by experts in Situation II.
FIG. 11 is a schematic diagram of the component placement sequences planned by the system for optimizing the process of the component retrieval and placement of the present disclosure in Situation II.
FIG. 12 is a schematic diagram of the component placement sequences planned by experts in Situation II.

### DETAILED DESCRIPTION OF PREFERRED EMBODIMENTS

Some specific embodiments according to the present disclosure will be described below; however, without departing from the spirit of the present disclosure, the present disclosure can still be practiced in various forms, and the protection scope of the present disclosure should not be construed as being limited to what is stated in the specification. In addition, unless otherwise indicated in the context, "a", "the" and similar terms used in the specification (especially in the appended claims) should be understood to include both the singular and the plural forms.

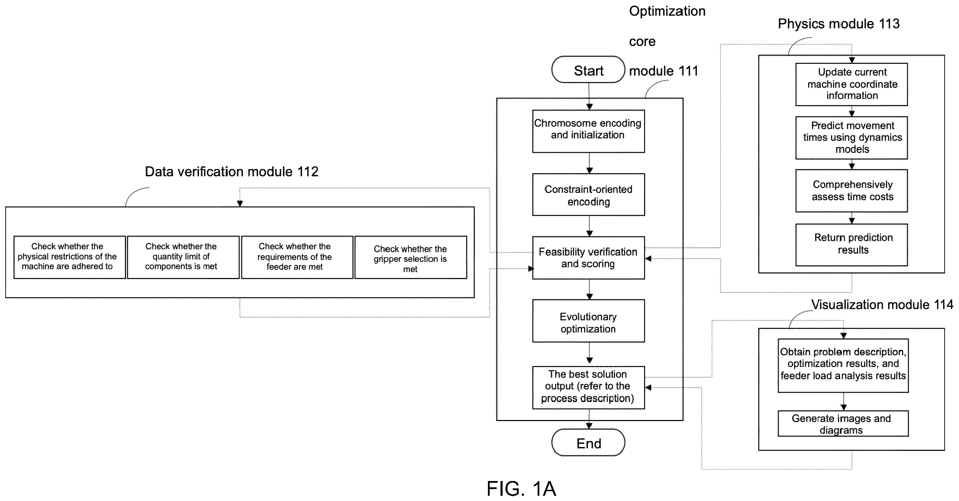

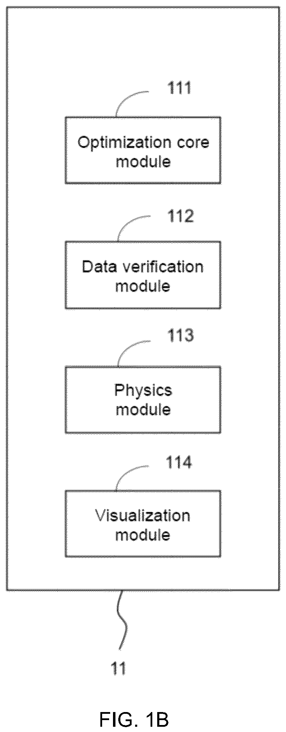

First, referring to FIGS. 1A and 1B, which are a flowchart of the system for optimizing the process of the component retrieval and placement of the present disclosure and a composition diagram of the optimization calculation module 11 in one embodiment of the system of the present disclosure, respectively. The present disclosure primarily discloses a system for optimizing the process of the component retrieval and placement. The system includes an optimization calculation module 11 configured to process multiple parameters and at least one constraint inputted by a user and output multiple final parameters representing optimized component retrieval and placement sequences and configurations based on results obtained via the processing, wherein the multiple parameters and the at least one constraint are associated with at least one of feeding mechanisms, pick-up mechanisms, types of components and component retrieval and placement sequences.

As illustrated in FIGS. 1A and 1B, the optimization calculation module 11 may include an optimization core module 111, a data verification module 112, a physics module 113, and a visualization module 114.

The optimization core module 111 is used to execute a genetic algorithm for optimization based on the multiple parameters and the constraints inputted by a user, wherein the multiple parameters and the constraints are associated with component retrieval and placement information, for example, are associated with at least one of feeding mechanisms, pick-up mechanisms, types of components and component retrieval and placement sequences. The genetic algorithm can find the best solution to a problem by simulating the process of natural selection. In this embodiment, the genetic algorithm may include: (i) encoding to randomly generate multiple chromosomes according to the multiple parameters, and the multiple chromosomes forming an initial chromosome pool; (ii) decoding the multiple chromosomes in the initial chromosome pool based on the at least one constraint; (iii) performing an evaluation on the decoded chromosomes to obtain fitness scores, and the evaluation includes a feedback of a penalty value when the decoded chromosomes violate the constraints; and (iv) selecting a chromosome with the smallest fitness score, and generating a chromosome that represents an optimized component retrieval and placement sequence and configuration through multiple iterations and selections.

In one embodiment, the above steps of encoding to randomly generate multiple chromosomes according to the multiple parameters means randomly selecting values for various variables such as the feeding mechanisms, the pick-up mechanisms, the types of components and the component retrieval and placement sequences as chromosome genes that encodes a chromosome to generate multiple chromosomes, ultimately forming an initial chromosome pool (or initial population).

In one embodiment, the at least one constraint may include a configuration sequence of the feeding mechanisms, correspondences between the pick-up mechanisms and the components to be picked, and a quantity limit of the components on the feeding mechanisms.

The data verification module 112 can be connected to the optimization core module 111 and configured to perform further evaluation on the decoded chromosomes to accelerate the convergence of the multiple iterations. The data verification module 112 verifies the feasibility of the decoded chromosomes in practical applications based on multiple factors. For example, the multiple factors may include: a) meeting the requirements of feeding mechanisms: checking whether the quantity of feeding mechanisms meets the user's needs; b) quantity limit: verifying whether the components provided by the feeding mechanisms are within the quantity limit of each single component; c) gripper selection : ensuring that the gripper is only used to grip components that can currently be gripped; d) compliance with physical restrictions: verifying whether the gripper number used for component retrieval and placement complies with the physical restrictions of the machine, such as the travel restriction.

The physics module 113 can be connected to the optimization core module 111 and configured to simulate an actual physical movement of a machine based on the decoded chromosomes to estimate a production cycle time for the decoded chromosomes. In one embodiment, an actuator model can be established in the physics module 113, which can simulate the dynamic movement of the machine, and the process may include: a) coordinate system maintenance: continuously tracking the coordinate information including all feeding mechanisms, grippers, standby points; b) dynamic simulation: utilizing physical models to simulate the movement of each axis, and estimating the movement time based on the movement distance; and c) time cost assessment: comprehensively considering the retrieval and placement process time corresponding to the entire chromosome, and feeding this information back to the optimization core module 111.

In one embodiment, the above steps of performing the evaluation on the decoded chromosomes to obtain the fitness scores are performed based on values of M, F, D, weight 1, and weight 2, wherein M represents a constant value for the evaluation, weight 1 represents the penalty value, F represents the quantity that exceeds the constraints, weight 2 represents a distance weight of the decoded chromosome, and D represents a distance; and when the fitness score is smaller, it means fewer constraints are violated, the distance is shorter, and the performance is better.

For example, if M is 1000, weight 1 is 10, and weight 2 is 1.5, and when these chromosomes do not violate any constraints:
the performance of a first group of chromosomes is: when a distance is 4.5, the fitness score should be: -1000+10×0+1.5×4.5 = -993.25,
the performance of a second group of chromosomes is: when a distance is 6.75, the fitness score should be: -1000+10×0+1.5×6.75 = -989.875,
since -993.25 is smaller than -989.875, the first group of chromosomes performs better, and from a distance perspective, the distance of the first group of chromosomes is also shorter;
when these chromosomes all violate one constraint:
   the performance of a third group of chromosomes is: when a distance is 2.5, the fitness score should be: -1000+10×1+1.5×2.5 = -986.25,
   the performance of a fourth group of chromosomes is: when a distance is 4.5, the fitness score should be: -1000+10×1+1.5×4.5 = -983.25,
   the performance of a fifth group of chromosomes is: when a distance is 7.75, the fitness score should be: -1000+10×1+1.5×7.75 = -978.375,
   since -986.25 is the smallest, the third group of chromosomes performs better, and from a distance perspective, the distance of the third group of chromosomes is also shorter.

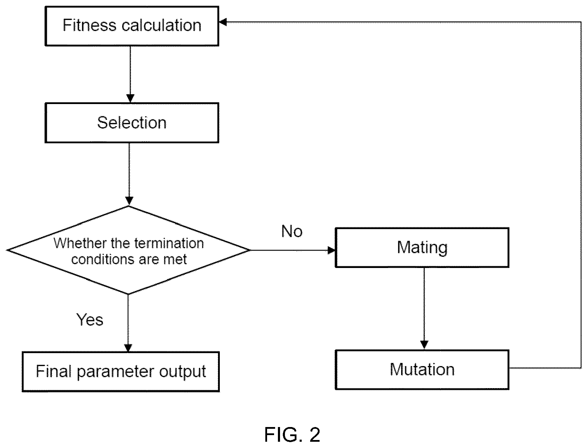

In a specific embodiment illustrated in FIG. 2, the above steps of selecting a chromosome with the smallest fitness score through multiple iterations and selections can be to select the chromosome with the smallest fitness score from the multiple chromosomes, wherein the chromosome with the smallest fitness score can refer to a chromosome with the lowest score or more than two chromosomes with the lower scores. Specifically, the steps may include selecting at least two chromosomes with the smallest fitness score based on the multiple parameters for mutual mating and mutation to generate new chromosome filial generations; performing the evaluation on the new chromosome filial generations to obtain the fitness scores, selecting at least two chromosome filial generations with the smallest fitness score (i.e., at least two chromosome filial generations with relatively low scores) from the new chromosome filial generations for another mutual mating and mutation to generate the next new filial generations of the chromosome filial generations; and taking a chromosome with the smallest value as the chromosome representing the optimized component retrieval and placement sequence and configuration when the number of the iterations exceeds a default value.

In one embodiment of the present disclosure, the default value can be a value between 10 and 100. For example, setting the default value to 100 means that the process of mating, mutation and producing filial generations can be repeated 100 times. After more than 100 times, the chromosome with the smallest fitness score is taken as the chromosome representing the optimized component retrieval and placement sequence and configuration. In other embodiments, the default value can be preferably 20 to 50, and can be more preferably 30, which represents that the process of mating, mutation and producing filial generations can be repeated 30 times, wherein the default value depends on the situation.

The visualization module 114 can be connected to the optimization core module 111 and configured to present the multiple final parameters in a diagram. In one embodiment, the optimization core module 111 can decode and output the chromosome representing the optimized component retrieval and placement sequence and configuration into a JSON format file, and then the visualization module 114 generates corresponding diagrams for the user based on the JSON format file. The visualization module 114 can provide multi-level visual presentation functions, and the process may include: a) visualization of problem description: clearly displaying the relative relationship between the pick-up mechanisms, the feeding mechanisms and placement points; b) optimization result display: visualizing the component retrieval and placement sequence of each travel and the pick-up mechanisms used; and c) feeding mechanism load analysis: providing visualization tools to evaluate the component retrieval balance of each feeding mechanism for inspection and reference by on-site experts.

In one embodiment, the multiple parameters may include, but are not limited to candidate positions of the feeding mechanisms, end point information of the pick-up mechanisms, positions of the placing points, physical limitations of actuators, correspondences between the pick-up mechanisms and the components, a configuration sequence of specific feeding mechanisms, and a quantity range of the specific components on the feeding mechanisms.

In one embodiment, the multiple final parameters may include, but are not limited to a total quantity of the feeding mechanisms, types of the components and configuration of the feeding mechanisms, configuration of the pick-up mechanisms and the feeding mechanisms, configuration of the pick-up mechanisms and the components, component retrieval and placement sequence, a predicted cycle time, a relation diagram of initial components and mechanisms, placing configuration and usage frequency distribution diagram of the feeding position, a path diagram of the component retrieval and placement, and a distribution diagram of a path length.

For example, the specific parameters inputted according to a specific situation in the present disclosure may include, but are not limited to the following: whether to specify the feeding arrangement; the name of the component (key value) and the upper limit (ub) and lower limit (lb) of the quantity of the feeding arrangement, for example, "A": { "lb":1, "ub":6}, representing that at least 1 and no more than 6 of material A should be placed; when the parameter "whether to specify the feeding arrangement" is true, the given placement is the main one, and the upper and lower limit are not be considered; whether to limit all end tools to only retrieve one type of components in all travels, and if it is false, the end tool can retrieve component A in a first travel and retrieve component B in a second pass; the limitation of the gripper for component retrieval, which only takes effect when the parameter "whether to specify the feeding arrangement" is true, and more than one possible selection can be listed, and thus the algorithm will ultimately select only one component for the gripper; the position, orientation and insertion component number of the insertion position relative to the product coordinate system, wherein "index" is the component number, "part" is the component to be inserted into the insertion position, "x, y, z" are the insertion point coordinates (based on the PCB origin, unit: mm), "theta" is the insertion rotation angle (degree), and "theta_pick" is the component retrieval rotation angle (degree).

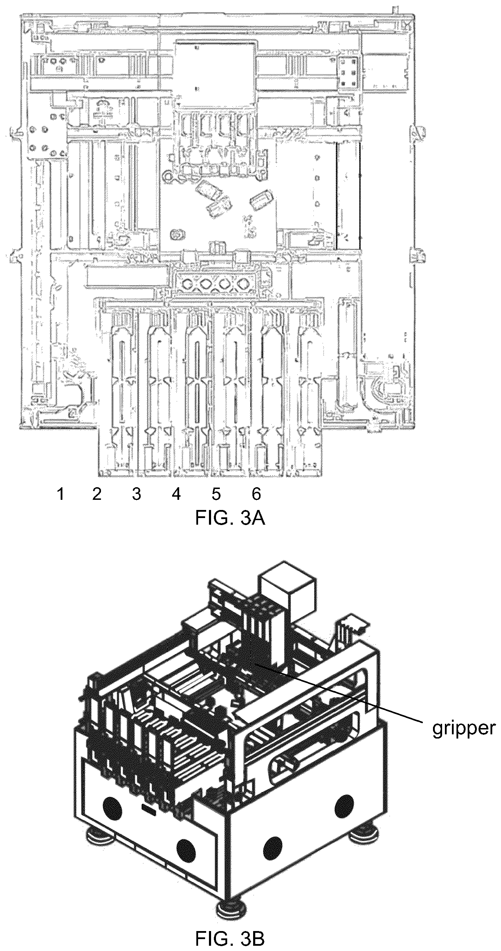

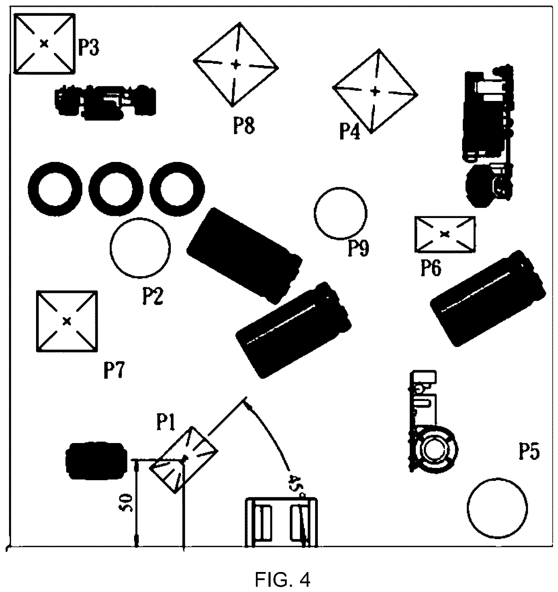

The optimization of PCB insertion production using the system for optimizing the process of the component retrieval and placement of the present disclosure is used as an illustration. Please refer to FIGs. 3A and 3B, the gripper is used as the pick-up mechanism and the feeder is used as the feeding mechanism in the embodiment. FIG. 3A is cross-sectional view of the insertion processing equipment, and FIG. 3B is a perspective view of insertion processing equipment, illustrating the sequence and configuration of the component retrieval and placement of the feeding mechanism and the pick-up mechanism. The insertion processing equipment is equipped with 6 feeding positions (e.g., feeder slots 1-6 shown in FIG. 3A), 4 grippers (shown in FIG. 3B) and 1 vision camera. The gripper should move to the position of the vision camera for pin abnormality detection after a round of component retrieval. In the embodiment, different types of components can be placed on the feeding position, but once selected, the feeding position can only be used for that type of components. The goal is to insert multiple types of components on a PCB into the position to be inserted and minimize the total cycle time to complete the PCB insertion work (as illustrated in FIG. 4).

The embodiment will be further described below in two situations. Each gripper is set for only one type of component retrieval in Situation 1, and one gripper is set for multiple types of component retrieval in Situation 2.

### Situation 1: retrieving a single type of components

This is a situation where the gripper can only retrieve one type of components. The same pick-up mechanism can only retrieve the same type of components between different travels. For example, if component A is retrieved for the first time, and component A should also be retrieved for the second time. In this situation, there are 24 insertion components, including 6 component A, 6 component B, and 12 component C. Detailed insertion component description is shown in Table 1.

**Table 1: insertion component description in Situation 1**

| Component number | Component type | The X-direction coordinate of the component on the PCB (X(mm)) | The Y-direction coordinate of the component on the PCB (Y(mm)) | Component placement angle on the PCB (Theta(Degree)) |
|---|---|---|---|---|
| P1 | A | -170.718 | 46.485 | 0 |
| P2 | A | -97.312 | 46.485 | 0 |
| P3 | A | -23.906 | 46.485 | 0 |
| P4 | A | -170.718 | 140.465 | 0 |
| P5 | A | -97.912 | 140.411 | 0 |
| P6 | A | -23.906 | 140.465 | 0 |
| P7 | B | -153.218 | 9.635 | 0 |
| P8 | B | -79.812 | 9.635 | 0 |
| P9 | B | -6.406 | 9.635 | 0 |
| P10 | B | -153.218 | 103.615 | 0 |
| P11 | B | -79.812 | 103.615 | 0 |
| P12 | B | -6.406 | 103.615 | 0 |
| P13 | C | -179.118 | 22.785 | 0 |
| P14 | C | -176.019 | 22.785 | 0 |
| P15 | C | -105.712 | 22.785 | 0 |
| P16 | C | -102.612 | 22.785 | 0 |
| P17 | C | -32.306 | 22.785 | 0 |
| P18 | C | -29.207 | 22.785 | 0 |
| P19 | C | -179.118 | 116.765 | 0 |
| P20 | C | -176.019 | 116.765 | 0 |
| P21 | C | -105.712 | 116.765 | 0 |
| P22 | C | -102.613 | 116.765 | 0 |
| P23 | C | -32.306 | 116.765 | 0 |
| P24 | C | -29.207 | 116.765 | 0 |

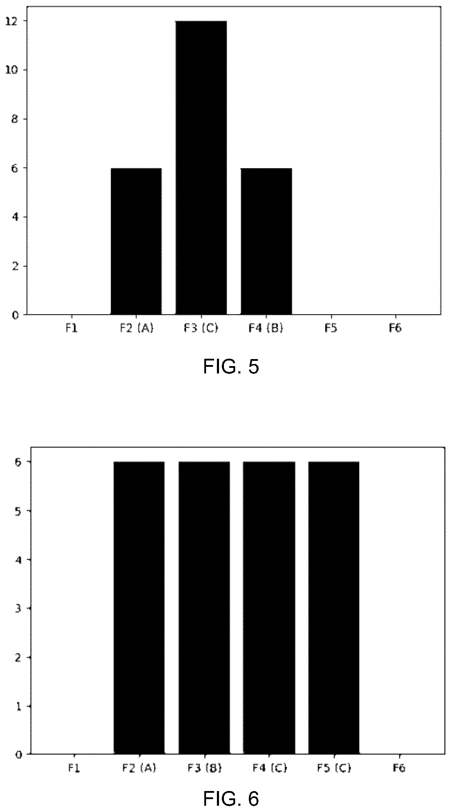

FIG. 5 is a chart of the feeder configuration planned by the system for optimizing the process of the component retrieval and placement of the present disclosure; a total of three feeding positions are used, where component A is placed in the second feeding position, component C is placed in the third feeding position, and component B is placed in the fourth feeding position. FIG. 6 is a chart of the feeder configuration planned by experts, where component A is placed in the second feeding position, component B is placed in the third feeding position, and component C is placed in the fourth feeding position, and component C is placed in the fifth feeding position. The X axis is the feeding position, and the Y axis is the number of uses. The feeding position with component placement will have the component type marked in brackets. First, whether the data inputted by the user is correct is checked; and then the optimization core module 111 is used in the system of the present disclosure to execute the genetic algorithm to find the optimized feeder configuration and component retrieval and placement sequence, and the data verification module 112 is used to estimate the feasibility of the previously obtained solution in practical applications to minimize the total cycle time for the feeder configuration and component retrieval and placement sequence.

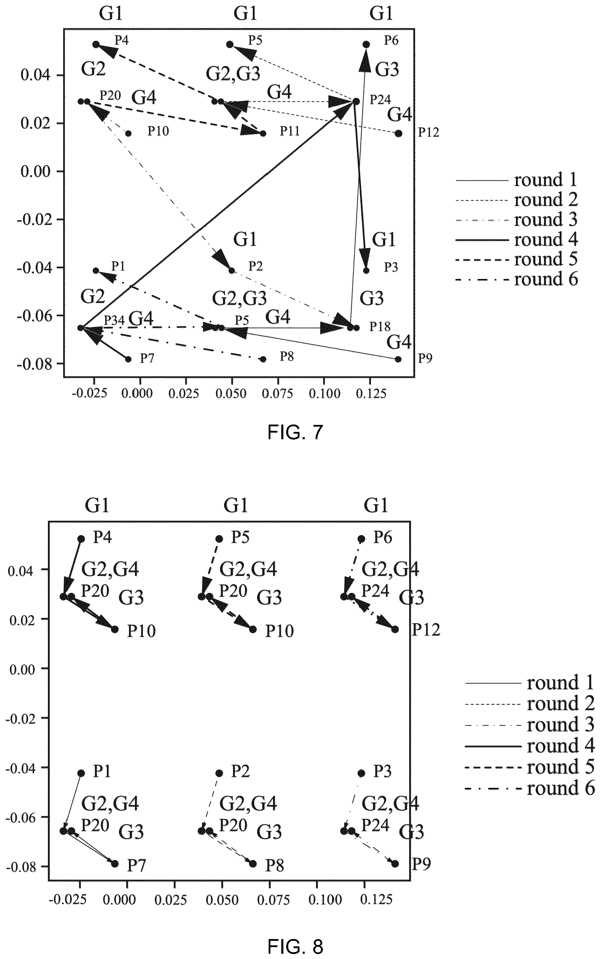

The movement of the insertion processing equipment is simulated by the physical module as shown in Table 2, and the results are presented in the diagrams by the visualization module 114 (as shown in FIGS. 7 and 8), wherein the right side of the component placement indicate which gripper is used to place components (i.e., P1, P2). The arrow marks of different colors represent the first to sixth travels respectively. The above results show that the planning time and expected cycle time of the system for optimizing the process of the component retrieval and placement of the present disclosure are both less than the expert's planning time and expected cycle time, and compared with the expert's results, one feed position is reduced and the movement time is reduced by 7%.

**Table 2: Comparison of the results in Situation 1**

| | The system for optimizing the process of the component retrieval and placement of the present disclosure | | | | Expert |
|---|---|---|---|---|---|
| Quantity of the feeder | 3 | 4 | 5 | 6 | 4 |
| Planning time | 2 min and 52 s | | | | 30 min |
| XY-axis cycle time (s) | 10.2 | 10.3 | 10.5 | 10.9 | 10.9 |

### Situation 2: retrieving various components

This is a situation where the gripper can only retrieve one type of components. The same pick-up mechanism can only retrieve the same type of components between different travels. For example, if component A is retrieved for the first time, and component A should also be retrieved for the second time. In this situation, there are 24 insertion components, including 6 component A, 6 component B, and 12 component C. Detailed insertion component description is shown in Table 3.

**Table 3: insertion component description in Situation 2**

| Component number | Component type | The X-direction coordinate of the component on the PCB (X(mm)) | The Y-direction coordinate of the component on the PCB (Y(mm)) | Component placement angle on the PCB (Theta(Degree)) |
|---|---|---|---|---|
| P1 | A | -170.718 | 46.485 | 0 |
| P2 | A | -97.312 | 46.485 | 0 |
| P3 | A | -23.906 | 46.485 | 0 |
| P4 | A | -170.718 | 140.465 | 0 |
| P5 | A | -97.912 | 140.411 | 0 |
| P6 | A | -23.906 | 140.465 | 0 |
| P7 | B | -153.218 | 9.635 | 0 |
| P8 | B | -79.812 | 9.635 | 0 |
| P9 | B | -6.406 | 9.635 | 0 |
| P10 | B | -153.218 | 103.615 | 0 |
| P11 | B | -79.812 | 103.615 | 0 |
| P12 | B | -6.406 | 103.615 | 0 |
| P13 | C | -179.118 | 22.785 | 0 |
| P14 | C | -176.019 | 22.785 | 0 |
| P15 | C | -105.712 | 22.785 | 0 |
| P16 | C | -102.612 | 22.785 | 0 |
| P17 | C | -32.306 | 22.785 | 0 |
| P18 | C | -29.207 | 22.785 | 0 |
| P19 | C | -179.118 | 116.765 | 0 |
| P20 | C | -176.019 | 116.765 | 0 |
| P21 | C | -105.712 | 116.765 | 0 |
| P22 | C | -102.613 | 116.765 | 0 |
| P23 | C | -32.306 | 116.765 | 0 |
| P24 | C | -29.207 | 116.765 | 0 |

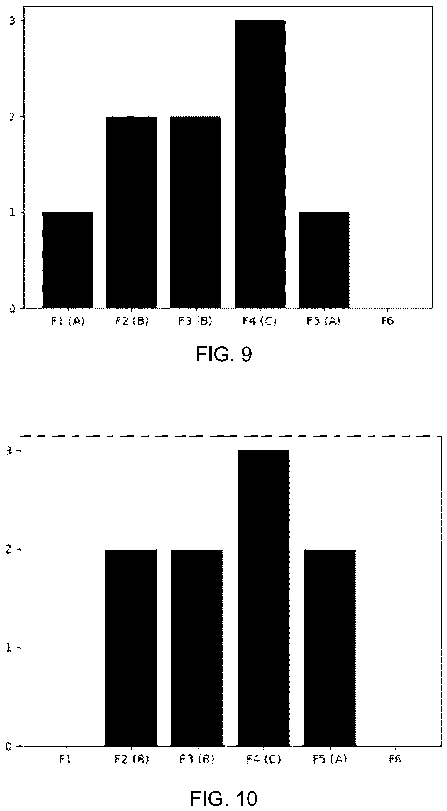

FIG. 9 is a chart of the feeder configuration planned by the system for optimizing the process of the component retrieval and placement of the present disclosure; a total of five feeding positions are used, where component A is placed in the first feeding position, component B is placed in the second feeding position, component B is placed in the third feeding position, component C is placed in the fourth feeding position, and component A is placed in the fifth feeding position. FIG. 10 is a chart of the feeder configuration planned by experts, where component B is placed in the second feeding position, component B is placed in the third feeding position, and component C is placed in the fourth feeding position, and component A is placed in the fifth feeding position. The X axis is the feeding position, and the Y axis is the number of uses. The feeding position with component placement will have the component type marked in brackets. First, as in Situation 1, whether the data inputted by the user is correct is checked; and then the optimization core module 111 is used in the system of the present disclosure to execute the genetic algorithm to find the optimized feeder configuration and component retrieval and placement sequence, and the data verification module 112 is used to estimate the feasibility of the previously obtained solution in practical applications to minimize the total cycle time for the feeder configuration and component retrieval and placement sequence.

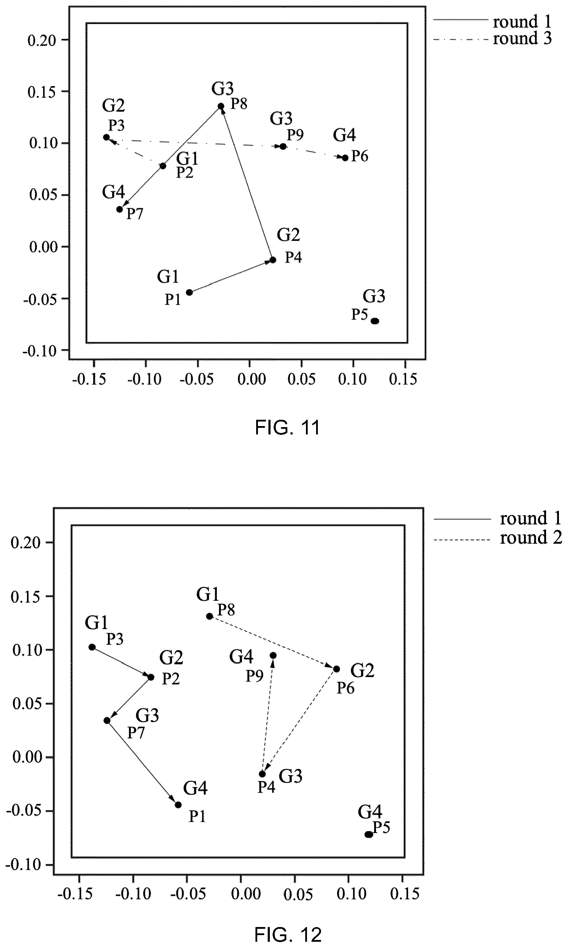

The movement of the insertion processing equipment is simulated by the physical module as shown in Table 4, and the results are presented in the diagrams by the visualization module 114 (as shown in FIGS. 7 and 8), wherein the right side of the component placement indicate which gripper is used to place components (i.e., P1, P2). The arrow marks of different colors represent the first to third travels respectively. There is no arrow-marked path in P5 of FIG. 11 and P5 of FIG. 12 since only one component is placed. The above results show that the planning time and expected cycle time of the system for optimizing the process of the component retrieval and placement of the present disclosure are both less than the expert's planning time and expected cycle time, and compared with the expert's results, the movement time is reduced by 29%.

**Table 4: Comparison of the results in Situation 2**

| | The system for optimizing the process of the component retrieval and placement of the present disclosure | Expert |
|---|---|---|
| Quantity of the feeder | 5 | 4 |
| Planning time | 1 min and 16 s | 10 min |
| XY-axis cycle time (s) | 3.7 | 5.2 |

Given the above, the system for optimizing the process of the component retrieval and placement of the present disclosure can effectively reduce cycle time in most situations, and significantly reduce the time required for expert planning and personnel to adjust machine configurations, thereby improving overall production efficiency and economic benefits.

## Claims

1. A system for optimizing a process of component retrieval and placement, **characterized in that** the system comprises an optimization calculation module (11) configured to process multiple parameters and at least one constraint inputted by a user and output multiple final parameters representing optimized component retrieval and placement sequences and configurations based on results obtained via the processing, wherein the multiple parameters and the at least one constraint are associated with at least one of feeding mechanisms, pick-up mechanisms, types of components and component retrieval and placement sequences, and the optimization calculation module (11) comprises:
an optimization core module (111), configured to execute a genetic algorithm;
wherein the genetic algorithm comprises (i) encoding to randomly generate multiple chromosomes according to the multiple parameters, and the multiple chromosomes form an initial chromosome pool; (ii) decoding the multiple chromosomes in the initial chromosome pool based on the at least one constraint; (iii) performing an evaluation on the decoded chromosomes to obtain fitness scores, and the evaluation includes a feedback of a penalty value when the decoded chromosomes exceed the at least one constraint;
and (iv) selecting a chromosome with the smallest fitness score, and generating a chromosome that represents the optimized component retrieval and placement sequence and configuration through multiple iterations and selections.

2. The system of claim 1, **characterized in that** the optimization calculation module (11) further comprises a data verification module (112) connected to the optimization core module (111) and configured to perform further evaluation on the chromosomes decoded by the optimization core module (111) to accelerate the convergence of iterations.

3. The system of claim 1, **characterized in that** the optimization calculation module (11) further comprises a physics module (113) connected to the optimization core module (111) and configured to simulate an actual physical movement of a machine based on the decoded chromosomes to estimate a production cycle time for the decoded chromosomes.

4. The system of claim 1, **characterized in that** the optimization calculation module (11) further comprises a visualization module (114) connected to the optimization core module (111) and configured to present the multiple final parameters in a diagram.

5. The system of claim 1, **characterized in that** the at least one constraint comprises a configuration sequence of the feeding mechanisms, correspondences between the pick-up mechanisms and the components to be picked, and a quantity limit of the components on the feeding mechanisms.

6. The system of claim 1, **characterized in that** the evaluation is performed based on M, F, D, weight 1, and weight 2 to obtain the fitness scores, and wherein M represents a constant value for the evaluation, weight 1 represents the penalty value, F represents the quantity that exceeds the at least one constraint, weight 2 represents a distance weight of the decoded chromosome, and D represents a distance.

7. The system of claim 1, **characterized in that** the multiple iterations and selections comprise selecting at least two chromosomes with the smallest value based on the multiple parameters for mutual mating and mutation to generate new chromosome filial generations; performing the evaluation on the new chromosome filial generations, selecting at least two chromosome filial generations with the smallest value for mutual mating and mutation to generate new filial generations of the chromosome filial generations; and taking a chromosome with the smallest value as the chromosome representing the optimized component retrieval and placement sequence and configuration when the number of iterations exceeds a default value.

8. The system of claim 1, **characterized in that** the multiple parameters comprise candidate positions of the feeding mechanisms, end point information of the pick-up mechanisms, positions of the placing points, and physical limitations of actuators.

9. The system of claim 8, **characterized in that** the multiple parameters further comprise correspondences between the pick-up mechanisms and the components, a configuration sequence of specific feeding mechanisms, and a quantity range of the specific components on the feeding mechanisms.

10. The system of claim 1, **characterized in that** the multiple final parameters comprise a total quantity of the feeding mechanisms, types of the components and configuration of the feeding mechanisms, configuration of the pick-up mechanisms and the feeding mechanisms, configuration of the pick-up mechanisms and the components, component retrieval and placement sequence, a predicted cycle time, a relation diagram of initial components and mechanisms, placing configuration and usage frequency distribution diagram of the feeding position, a path diagram of the component retrieval and placement, and a distribution diagram of a path length.

11. A method for optimizing a process of component retrieval and placement, **characterized in that** the method is executed by an optimization calculation module (11) of a system for optimizing a process of component retrieval and placement as claimed in any one of claims 1 to 10.
